(19) Europäisches Patentamt
European Patent Office
Office européen des brevets

(11) Publication number : **0 310 622 B1**

(12) # EUROPEAN PATENT SPECIFICATION

(45) Date of publication of patent specification : **15.04.92 Bulletin 92/16**

(51) Int. Cl.$^5$ : **A61K 31/00,** C07C 69/608, C07C 69/612, C07C 69/614

(21) Application number : **87904184.6**

(22) Date of filing : **10.06.87**

(86) International application number : **PCT/US87/01371**

(87) International publication number : **WO 87/07599 17.12.87 Gazette 87/28**

(54) **ANTI-INFLAMMATORY COMPOSITIONS.**

Divisional application 91111471.8 filed on 10/06/87.

(30) Priority : **11.06.86 US 872812**

(43) Date of publication of application : **12.04.89 Bulletin 89/15**

(45) Publication of the grant of the patent : **15.04.92 Bulletin 92/16**

(84) Designated Contracting States : **AT BE CH DE FR GB IT LI LU NL SE**

(56) References cited :
**Journal of the American Oil Chemists' Society, volume 61, no. 7, July 1984 G.T. Marshall et al.: "Short-chain phorbol ester constituents of croton oil", pages 1220-1225**

(73) Proprietor : **ALDER RESEARCH CENTER CORPORATION
165 New Boston Street
Woburn, MA 01801 (US)**

(72) Inventor : **DRIEDGER, Paul, E.
30 Symmes Street
Winchester, MA 01890 (US)**

(74) Representative : **Holdcroft, James Gerald, Dr. et al
Graham Watt & Co., Riverhead
Sevenoaks, Kent TN13 2BN (GB)**

EP 0 310 622 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Protein kinase C is an enzyme found in nearly all animal tissues and animal cells that have been examined. Its identity is generally established by its ability to phosphorylate proteins when adenosine triphosphate, calcium ions and phospholipid cofactors are present, with greatly reduced activity when these cofactors are absent. Protein kinase C activity is substantially stimulated by certain 1,2-sn-diacylglycerols that bind specifically and stoichiometrically to a recognition site on the enzyme. Stimulation of protein kinase C by these diacylglycerols has been shown to be an important physiological event that mediates the actions of a wide variety of hormones, neurotransmitters, and other biological control factors such as histamine, vasopressin, alpha-adrenergic agonists, dopamine agonists, muscarinic cholinergic agonists, platelet activating factor, etc. [see Y. Nishizuka, Nature 308 693-698 (1984) and Science 225 1365-1370 (1984) for reviews].

The biological role of protein kinase C is also of great interest because of the discovery that certain very powerful tumor promoting substances activate this enzyme by binding specifically and with very high affinity to the diacylglycerol binding site on the enzyme. In addition to diacylglycerols, there are at present five other known classes of compounds that bind to this site, including diterpenes such as the phorbol esters, indole alkaloids (indolactams) such as the teleocidins, lyngbyatoxin, and indolactam V, polyacetates such as the aplysiatoxins and oscillatoxins, certain derivatives of diaminobenzyl alcohol, and the macrocyclic lactones of the bryostatin class. The phorbol esters have long been known as powerful tumor promoters, the teleocidins and aplysiatoxins are now known to have this activity, and it appears likely that additional classes of compounds will be found to have the toxic and tumor promoting activities associated with the capability to bind to the diacylglycerol site of protein kinase C and thus activate the enzyme. Other toxicities of these agents when administered to animals include lung injury and profound changes in blood elements, such as leukopenia and neuropenia.

In addition to potent tumor promoting activity, these six classes of compounds, collectively referred to as the "phorboids", display a vast range of biological activities, as would be expected from the widespread distribution of their target enzyme. Some of these activities, like tumor promotion, indicate the involvement of protein kinase C in important normal or pathological processes in animals. Thus, the phorboids are potent skin inflammatory agents, cause smooth muscle contraction in several tissues, alter immune system function and can be used to cause a variety of other normal or pathological responses. Related disease states such as the development of cancer, the onset and/or maintenance of inflammatory disease, the role of vasoèconstriction in hypertension, the role of broncho-constriction in asthma, the role of cholinergic, adrenergic, and dopaminergic synapses in diseases of the central/peripheral nervous systems, may be mediated in vivo by the stimulation of protein kinase C by diacylglycerols, the latter being generated in the cell by pathological agents or conditions.

In analyzing the activity of a pharmaceutical or other bioactive compound, it is useful to consider two properties: the efficacy, defined as the capability to elicit a full or partial biological result, such as complete displacement of a ligand from its receptor site or the complete inhibition of inflammation or edema caused by a standard stimulus; and the potency, defined as that amount or concentration of drug that causes 50% of the full response (often abbreviated as the $ED_{50}$). It is frequently the case within a given class of pharmaceutical agents that individual members of the class all have equal efficacy, i.e. they each can generate a full biological effect, but they show differening potencies. Thus, the structural modifications within such a class affect only the amount necessary to achieve a given result, and the modified compounds otherwise have generally the same central biological characteristic. There may also be differences between members of such a class as regards properties other than the central biological characteristic; for example, members of the class might differ in side effects or toxicity.

Well-known pharmaceuticals that have been in extensive use for years or decades show a wide range of optimal therapeutic potencies. Aspirin, for example, is often taken in multi-gram amounts per day for treatment of inflammation or arthritis, and detailed analyses of its mechanism of action in vitro show that a concentration in the millimolar range is required. In contrast, steroid-based topical anti-inflammatory compounds such as fluocinolone acetonide are many thousand-fold more potent, and, beyond this, some oral contraceptive agents are prescribed in daily doses in the microgram range. Thus, although high potency is generally advantageous for a pharmaceutical, it is not an absolute requirement.

Several thousand of the high skin-inflammatory and tumor-promoting phorboids have been reported in the literature, including numerous examples on which minor chemical modifications have been made [see Evans and Soper, Lloydia 41 193-233 (1978) and references cited therein]. The structures of these phorboids can be compared, and their activities for inflammation and tumor promotion can be analyzed from the perspective of efficacy and potency. The structures of the different classes of phorboids vary quite markedly from one to the other class yet widespread testing of their biological activities has shown that these classes have generally very similar biological properties. In particular, the thousands of known phorboids of the highly potent diterpene,

indolactam, and polyacetate classes appear to have, with very minor exceptions, virtually identical efficacies as skin irritants and tumor promoters [T. Sugimura, Gann 73 499-507 (1982)]. The exceptions involve a few compounds that have a short duration of irritant activity and/or manifest diminished tumor promoting activity, perhaps due to toxicity or secondary parameters such as differing metabolic destruction rates.

In contrast to the essentially equal efficacies among the vast majority of phorboids, their relative potencies cover a wide range, as measured in inflammation and promotion tests and as measured in numerous other in vivo and in vitro systems. Example compounds can be found in the diterpene, indolactam, and polyacetate classes that have nearly equal, very high potencies. At the same time there are compounds in each of these classes which embody significant structural changes that do not diminish efficacy but do result in potency decreases of 10-fold to 100,000-fold or more [see, for example, Driedger and Blumberg, Cancer Res. 37 3257-3265 (1977), Cancer Res. 39 714-719 (1979)]. Thus, all these compounds appear to be capable of achieving generally the same biological results, and merely differ in the amount which must be used to obtain a given result.

In vitro measurements of biochemical properties provide an even more sensitive method for comparing the properties of the various phorboids. For example, using a radioactively labeled phorboid such as [$^3$H]phorbo1 12,13-dibutyrate or [$^3$H]lyngbyatoxin, one can measure the potency of a test compound as a competitive ligand for the diacylglcerol binding site on protein kinase C. Alternatively, one can measure the ability of a given phorboid to stimulate the protein kinase C-mediated incorporation of radioactive phosphate from [$^{32}$P]adenosine triphosphate into a standard acceptor substrate such as histone H1. Tests of this nature reveal a difference in potency between given phorboid agonists of as much as 10,000,000-fold or more [Dunn and Blumberg, Cancer Res. 43 4632-4637 (1983), Table 1].

These basic data regarding the phorboid agonists are an important consideration because they underscore the concept that the structural differences among these previously known phorboids, especially the diterpenes, indolactams, polyacetates, and bryostatins, generally do not affect their efficacies as toxic agonists, and indeed a wide variety of structural changes are tolerated in this regard. Such changes generally alter potency only and do not provide agents with therapeutic utility, since they retain their toxicity.

Some minor changes in phorboid structure are known to result in inactive compounds, such as a stereochemical change from 4-beta to 4-alpha in the phorbol series, and indeed some of the diterpene skeleton structures carry hydroxy groups that must be esterified in order for inflammatory activity to be observed. However, these inactive compounds are quite few in number among the known phorboids, and no therapeutic utility has been demonstrated for them.

The phorbol esters, indolactams, polyacetates, diaminobenzyl alcohols, and bryostatins are generally found in plants, molds, and algae, or are synthetic in origin. Although they are found in many parts of the world, normal human contact with them is thought to be low. In contrast, the diacylglycerols are part of the functioning of virtually every type of animal cell except erythrocytes of some species, and thus the undesirable activation of protein kinase C by the diacylglycerols may have a very widespread role in human diseases.

Thus, new compounds, capable of blocking the activation of protein kinase C by acting as specific pharmacological antagonists of the diacylglycerols, would be valuable agents in the prevention and treatment of a wide variety of diseases in animals and humans.

There may be several different forms of protein kinase C each having different biological roles. The stimulation of one form can lead to undesirable results such as inflammation or the development of cancer, while stimulation of another form of the enzyme might produce beneficial effects, such as the abrogation of inflammation or the secretion of useful bioregulatory factors such as hormones and interferons.

Moreover, the exact correspondence between diacylglycerol binding sites and protein kinase C has not been fully explored, and there may be several different such binding sites with differential affinities for diacylglycerols and other phorboids. Indeed there is published evidence for several distinct classes of phorboid binding sites in various tissues and cell types (Dunn and Blumberg, op. cit.). However, in this study, even the ligands showing the clearest differences in affinity for these distinct classes are only selective by a factor of 10-100 dissociation constant. Thus, other new compounds, capable of selectively activating one useful, but not another, deleterious, diacylglycerol target site, would also be valuable agents for the prevention or treatment of inflammation.

Earlier efforts to use the previously known phorboids themselves or to modifiy of the structures of the known phorboids, have not been successful in producing useful compounds.

It has been known for some time that several of the toxic, inflammatory and tumor-promoting compounds such as phorbol 12-tigliate 13-decanoate, mezerein, lyngbyatoxin and aplysiatoxin have antileukemic activity in mouse model tests. However, these compounds are all extremely toxic and are cancer suspect agents, thus eliminating them from consideration as human therapeutic agents.

Ganong et al. (Proc. Nat. Acad. Sci. 83 1184-1188 (1986)] tested a series of diacylglycerols and found no antagonistic activity in that series against the standard agonist 1,2-dioctanoylglycerol. It is of particular note

that several compounds tested in this work were modified in the hydroxymethyl portion of the diacylglycerol molecule, and these modifications produced only a loss of activity or a weakened activity that was not distinguishable from the agonist activity of 1,2-dioctanoylglycerol itself. These hydroxymethyl-modified compounds were not antagonists in these tests and no utility was found. Similarly, Thielmann and Hecker [Forsch. Krebsforsch. Vol. VII, pp. 171-179 (1969), New York: Schattauer] found only a complete loss of biological activity in their study when the hydroxy group of the hydroxymethyl on phorbol 12,13 didecanoate was replaced with hydrogen or chlorine. Schmidt and Hecker (H. Lettre and G. Wagner (eds.), Aktuelle Probleme aus dem Gebiet der Cancerologie, Vol. III, 3rd Heidelberg Symposium, pp. 98-108. Berlin: Springer Verlag, 1971.] also found that oxidation of the hydroxymethyl of phorbol 12,13 didecanoate to a carboxylic acid caused complete loss of activity in the assays used.

The hydroxymethyl group of the known phorboids (discussed in more detail in Summary of the Invention) has been thought to be required for biological activity, as detailed by Hecker (Carcinogenesis, Vol. 2, eds. Slaga, Sivak and Boutwell, Raven Press, New York, 1978, pp. 11-48 and references cited therein). Indeed, it is stated therein that the replacement of the 20-hydroxyl in a phorbol ester "results in complete loss of niological activity". In another study, replacement of the hydroxy group of the hydroxymethyl (located at carbon 14) by chlorine or hydrogen in indolactam V gave rise to compounds with agonist activity weaker than but otherwise not distinguished from the agonist activity of the very toxic teleocidin class of tumor promoters (Irie et al., Int. J. Cancer 36 485-488 (1985)]. Thus no utility was found.

Schmidt and Hecker (Carcinogenesis, Vol. 7, ed. by E. Hecker et al., Raven Press, New York, 1982, pp. 57-63) studied the abilities of a series of diterpene phorboids to inhibit tumor promotion by the standard phorboid agonist tumor promoter phorbol 12-myristate 13-acetate (PMA). They found that, at low doses, some short-chain ester derivatives of phorbol were able to block the tumor promotion by PMA. However, all of the compounds that were active as antagonists at low doses are also very efficacious skin irritants themselves at higher doses and most of them are also known to have tumor promoting activity. Thus, these short-chain esters still have toxic inflammatory and tumor promoting activity and thus have no therapeutic value. In this publication it was also noted that a phorbol 12-ester, namely phorbol 12-myristate, was without activity as an antagonist of PMA-induced tumor promotion. Theilmann and Hecker (op. cit.) also found phorbol 12-decanoate and phorbol 12-myristate to be inactive in the functional tests used, and stated that the 13-OH must be esterified to obtain active compounds.

## Summary of the Invention

This invention pertains to diterpenoid phorboid derivatives which lack the toxic properties of previously available diterpenoid phorboids. These diterpenoid phorboid derivatives of very diverse structures have utility as anti-inflammatory agents, anti-cancer and anti-leukemic agents, anti-asthmatic and anti-hypertensive agents, as modulators of in vivo immune function, as stimulators of the production of lymphokines such as interferon and the interleukins, as stimulants of hair growth on bald or partially bald scalp, as central nervous system pharmaceuticals for several pathological conditions, and as xenobiotics for achieving the control of parasites.

The structures of some of the previously known diterpenoid phorboids are shown below.

TYPICAL DITERPENE-TYPE PHORBOID AGONISTS

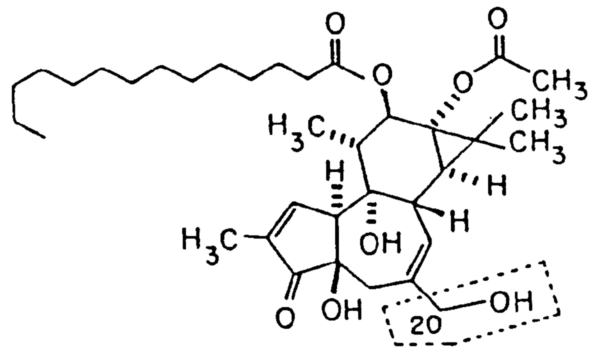

Phorbol 12-Myristate 13-Acetate (PMA)

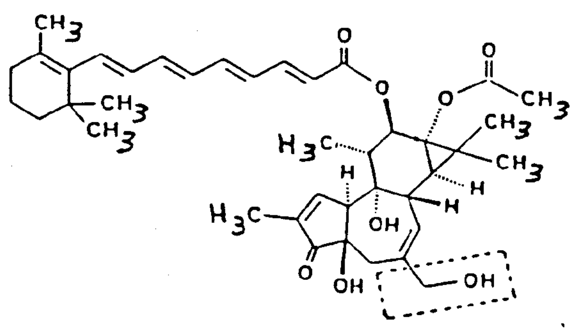

Phorbol 12-Retinoate 13-Acetate

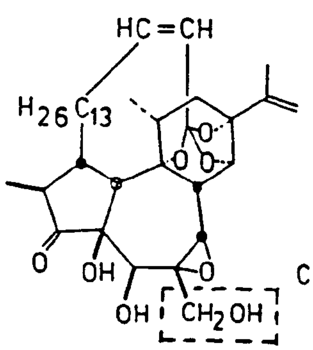

Synaptolepsis Factor $K_1$

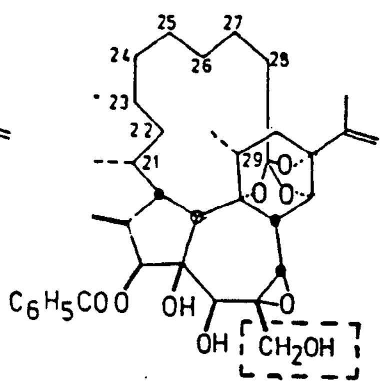

Pimelea Factor $P_2$

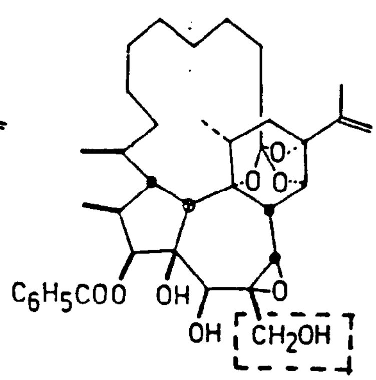

Daphnopsis Factor $R_6$

TYPICAL DITERPENE-TYPE PHORBOID AGONISTS (cont.)

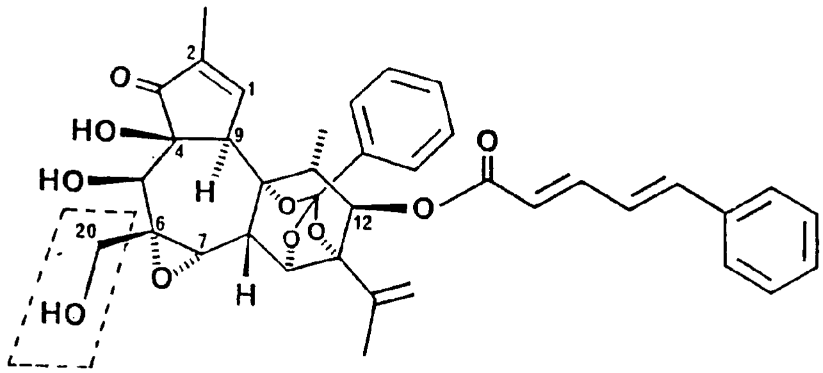

MEZEREIN

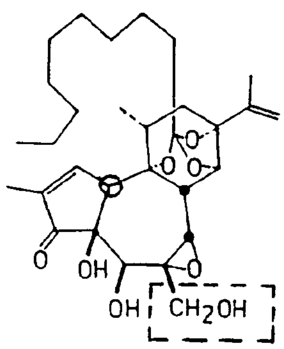

Simplexin

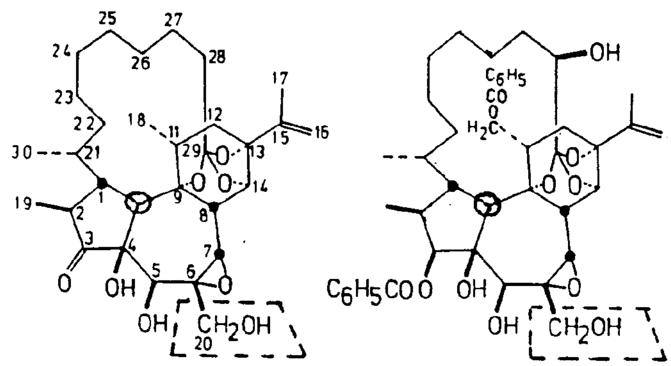

Pimelea Factor $S_7$

Gnidimacrin

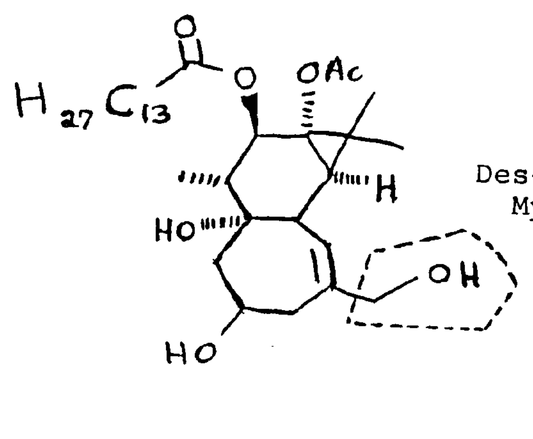

Des-(Ring A)-phorbol 12-Myristate 13-Acetate

It can be seen that the diterpenoid phorboids depicted have exceeding diverse structural elements, with one prominent exception, namely that each contains a hydroxymethyl or 1-hydroxyethyl group (indicated by the dashed-line boxes in each structure). In each case the phorboid depicted is among the most potent of its particular structural class, and among the classes the diterpenes, indolactams and polyacetates have members of especially high potency.

It has now been found that compounds of the diterpene class, with the usual hydroxymethyl or 1-hydroxyethyl group intact and having at least on substituent other than hydrogen at $C_{12}$ and a hydroxy, amino, thiol,

hydroxymethyl, mercaptomethyl, aminomethyl, 2-hydroxyethyl, carboxy, unsubstituted carboxamido, or unsubstituted aminocarbonyloxy group in the alpha configuration at $C_{13}$, display useful properties as anti-inflammatory agents. In this very unusual case a minor change in the diterpene moiety at a location other than the hydroxymethyl leads to a very substantial and useful change in biological properties. This is in contrast to the belief that diterpenes such as phorbol 12-decanoate, 12-dodecanoate and 12-myristate are biologically inactive, and it is especially in contrast to the usual association of the hydroxymethyl or 1-hydroxyethyl groups with pro-inflammatory activity.

Accordingly the invention provides a compound for use in medicine, the compound being of the general formula (I):

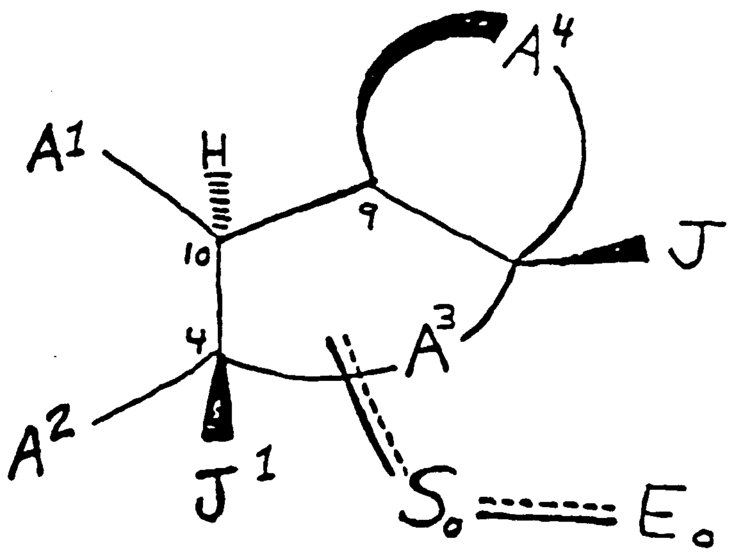

(I)

in the form of an individual isomer, an isomer mixture, a racemate or optical antipode, or a pharmaceutically acceptable salt thereof;
wherein

A1 and A2 may be individually selected from hydrogen and a straight chain or branched chain, cyclic or acyclic, saturated, unsaturated and/or aromatic carbon- and/or heteroatom-containing substituent having not more than 34 carbon atoms, not more than 24 halogen atoms and not more than 6 heteroatoms selected from oxygen, nitrogen and sulfur; or,

$A^1$ and $A^2$ taken together complete a 5- or 6-membered carbocyclic or heterocyclic ring, optionally substituted by one or more straight chain or branched chain, cyclic or acyclic, saturated, unsaturated, and/or aromatic carbon- and/or heteroatom-containing groups, which groups taken together contain a total of not more than 30 carbon atoms, not more than 24 halogen atoms and not more than 6 heteroatoms selected from oxygen, nitrogen, and sulfur;

$A^3$ is a three atom chain which completes a 7-membered carbocyclic ring optionally substituted by one or more straight chain or branched chain, cyclic or acyclic, saturated, unsaturated, and/or aromatic carbon- and/or heteroatom-containing groups, which groups taken together, excluding SoEo, contain not more than 7 carbon atoms, not more than 4 halogen atoms, and not more than 5 heteroatoms selected from oxygen, nitrogen and sulfur; provided that, excluding $S_oE_o$, the middle carbon atom of $A^3$ is not substituted by hydroxymethyl, hydroxyethyl or 2-hydroxy-2-propyl;

$A^4$ completes a 6- membered carbocyclic ring (connected in the beta configuration to carbon atom 9), optionally substituted by one or more straight chain or branched chain cyclic or acyclic, saturated, unsaturated and/or aromatic carbon- and/or heteroatom-containing groups, linked to $A^4$ by single or double bonds, the group or groups optionally completing one or two additional rings by themselved and/or one or two additional rings by themselves and/or one to three additional rings when taken together with A1, A2, a ring formed by A1 and A2 together, and/or a bond to carbon atom 9, which groups taken together include not more than 40 carbon atoms, not more than 24 halogen atoms, and not more than 10 heteroatoms selected from oxygen, nitrogen, and sulfur; and wherein $A^4$ carries at least one substituent other than hydrogen or hydroxy at $C_{12}$ and a hydroxy,

amino, thiol, hydroxymethyl, mercaptomethyl, aminomethyl, 2-hydroxyethyl, carboxy, unsubstituted carboxamido, or unsubstituted aminocarbonyloxy group in the alpha configuration at $C_{13}$; $J^1$ is selected from hydrogen, fluoro, chloro, hydroxy, amino, mono- or di-loweralkylamino, methyl, ethyl, vinyl, ethynyl, propargyl, cyano, methoxy, ethoxy, trifluoromethyl, 2-hydroxyethyl, 2-hydroxypropyl, 3-hydroxypropyl, acetoxy, propanoyloxy, acetyl, propanoyl, hydroxyacetyl, 2-hydroxypropanoyloxy, 3-hydroxypropanoyl, acetamido, propanamido, hydroxyacetamido, 2-hydroxypropanamido, or 3-hydroxypropanamido, (each of which must be situated in the beta position), or $J^1$ taken together with A1, A2, or a ring formed by A1 together with A2 completes a 3- to 7-membered substituted or unsubstituted carbocyclic or heterocyclic ring;

J2 is selected from hydrogen, methyl, ethyl, hydroxymethyl, hydroxyethyl, vinyl, ethynyl, allyl, propargyl, n-propyl and iso-propyl;

$S_oE_o$ taken together comprise hydroxymethyl, 1-hydroxyethyl or 2-hydroxy-2-propyl.

The compound of the formula (I) suitably has a substituent SoEo on the middle carbon of $A^3$, wherein SoEo comprises $-CH_2OH$, $CH(CH_3)OH$ or $C(CH_3)_2OH$. In particular, the compound of the formula (I) may be a phorbol 12-ester.

Examples of the formula (I) are:

phorbol 12-[4-(9,10-dihydrophenanthrene-2)-butyrate];
phorbol 12-(2,4-difluorophenylacetate);
phorbol 12-[3,5-bis(trifluoromethyl) benzoate]:
phorbol 12-[3,5-bis(trifluoromethyl)-phenylacetate;
phorbol 12-(4-n-hexylbenzoate);
phorbol 12-(3,5-dimethoxyphenylacetate)
phorbol 12-(4-phenylbenzoate); and
phorbol 12-myristate

The invention also provides novel compounds per se having the general formula (I) as hereinbefore defined but excluding phorbol 12-n-alkanoyl esters, phorbol 12-alkenoyl esters, phorbol 12-benzoate and phorbol 12-(12′-N-dansylaminododecanoate).

In a further aspect, the invention provides the use of a compound, novel or known, of the formula (I) for the manufacture of a medicament for treating inflammatory conditions.

Also provided is a pharmaceutical composition containing a compound of the formula (I) and a pharmaceutically acceptable carrier. Excluded from this definition are the simple solutions of known diterpene phorboids discussed in the introductory portion of this application, for example as disclosed in the Schmidt and Hecker article (Carcinogenesis, Vol. 7, Ed. by E Hecker et al, Raven Press, New York, 1982 pp 57-63. Thus simple solutions of the known diterpenoid phorbol-12-esters wherein the carrier is dimethylsulfoxide, acetone, or methanol or ethanol of greater than 80% concentration in water, are excluded from the term "pharmaceutical compositions".

The compounds of this invention have been found to possess valuable pharmacological properties. They block inflammation, block proliferation of cancer cells, and induce production of thrombolytic activity in human and veterinary medicine. These effects can be demonstrated, for example, by use of standard mouse ear inflammation tests by established agonists such as PMA and the ionophore A23187, by the inhibition of proliferation of human cancer cells in culture by induction of differentiation, and by measurement of fibrinolytic activity in cultured cells.

These compounds also show selective effects as antagonists or protein kinase C in some cases, as noninflammatory agonists for protein kinase C in other cases, and as selective ligands for protein kinase C and/or for phorboid receptors.

Thus, these compounds can be used as agents for the abrogation of pathophysiological conditions and disease states in applications such as anti-inflammatory, anti-psoriatic, anti-cancer, anti-ulcer, anti-hypertensive, anti-asthma, anti-arthritic, anti-autoimmune, anti-nociceptive, anti-secretory, anti-parasitic, anti-amoebic, anti-HTLV-III/LAV viral replication, and any other application in which pathological involvement of protein kinase C is found.

Furthermore, the non-toxic agonists among the compounds of this invention may be used to achieve desired physiological results such as interferon release, interleukin induction, tumor necrosis factor production, immune system stimulation and/or reconstitution, insulin secretion, insulinomimetic activity, acceleration of wound healing, improvement in central nervous system functions such as memory and learning and abrogation of the symptoms or progress of Alzheimer's disease, and any other application for which desirable actions of protein kinase C are found.

As receptor subtype- and/or protein kinase C substype-selective ligands, the compounds of this invention also have very valuable application as experimental agents for research into the role of protein kinase C and/or phorboid receptors in important biological processes and in human and veterinary diseases. Thus, their value

extends to their use as pharmacological tools for in vitro and in vivo research, in a manner similar to the important roles that selection agonists and antagonists have played in the studies of the mechanism of action of adrenergic, dopaminergic, opiate, benzodiaepine, cholinergic, and serotoninergic receptor systems, among others.

In addition, the compounds can be used in in vitro diagnostics (e.g., in an assay for protein kinase C). They are also useful as intermediates in the production of other drugs, e.g., as described in the present invention.

The compounds of this invention are generally administered to animals, including but not limited to fish, avians, and mammals including humans.

The pharmacologically active compounds of this invention can be processed in accordance with conventional methods of galenic pharmacy to produce medicinal agents for administration to patients, e.g., mammals including humans.

The compounds of this invention can be employed in admixture with conventional excipients, i.e., pharmaceutically acceptable organic or inorganic carrier substances suitable for parenteral, enteral (e.g., oral) or topical application which do not deleteriously react with the active compounds. Suitable pharmaceutically acceptable carriers include but are not limited to water, salt solutions, alcoholcs, gum arabic, vegetable oils, benzyl alcohols, polyethylene glycols, gelatine, carbohydrates such as lactose, amylose or starch, magnesium stearate, talc, silicic acid, viscous paraffin, perfume oil, fatty acid esters, hydroxy methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds. They can also be combined where desired with other active agents, e.g., enzyme inhibitors, to reduce metabolic degradation.

For parenteral application, particularly suitable are injectable, sterile solutions, preferably oily or aqueous solutions, as well as suspensions, emulsions, or implants, including suppositories. Ampoules are convenient unit dosages.

For enteral application, particularly suitable are tablets, dragees, liquids, drops, suppositories, or capsules. A syrup, elixir, or the like can be used wherein a sweetened vehicle is employed.

Sustained or directed release compositions can be formulated, e.g., liposomes or those wherein the active compound is protected with differentially degradable coatings, e.g., by microencapsulation, multiple coatings, etc. It is also possible to freeze-dry the new compounds and use the lyophilizates obtained, for example, for the preparation of products for injection.

For topical application, there are employed as nonsprayable forms, viscuous to semi-solid or solid forms comprising a carrier compatible with topical application and having a dynamic compatible with topical application and having a dynamic viscosity preferably greater than water. Suitable formulations include but are not limited to solutions, suspensions, emulsions, creams, ointments, powders, liniments, salves, aerosols, etc., which are, if desired, sterilized or mixed with auxiliary agents, e.g., preservatives, stabilizers, wetting agents, buffers or salts for influencing osmotic pressure, etc. For topical application, also suitable are sprayable aerosol preparations wherein the active ingredient, preferably in combination with a solid or liquid inert carrier material, is packaged in a squeeze bottle or in admixture with a pressurizied volatile, normally gaseous propellant, e.g., a freon.

Generally, the compounds of this invention are dispensed in unit dosage form comprising 0.01 to 1000 mg in a pharmaceutically acceptable carrier per unit dosage. They are incorporated in topical formulations in concentrations of about 0.01 to 10 weight percent.

It will be appreciated that the actual preferred amounts of active compound in a specific case will vary according to the specific compound being utilized, the particular compositions formulated, the mode of application, and the particular situs and organism being treated. Dosages for a given host can be determined using conventional considerations, e.g., by customary comparison of the differential activities of the subject compounds and of a known agent, e.g., by means of an appropriate, conventional pharmcological protocol.

Starting materials for the synthesis of the compounds of this invention may be obtained from any of a wide variety of natural sources, as described in the literature for the diterpenes (R. Schmidt and E. Hecker, Fortschritte D. Chemie Organischer Naturstoffe 31 377-467 (1974) and F. J. Evans and C. J. Soper, Lloydia 41 193-233 (1978), and references cited therein). Furthermore, the diterpene compounds are available by synthesis de novo (See P.A. Wender, Am. Chem. Soc. National Meeting, Chicago, IL, 9 September 1985, Abstract #7, and P.A. Wender et al. Proc. Nat. Acad. Sci. 83, 4214-4218, 1986).

The use of the methods of total synthesis as described in the literature cited above, with obvious adaptations, permits specific modifications of the parent structures in the diterpenes by established techniques in the art of synthetic chemistry, to obtain modified parent structures having anti-inflammatory activity, and/or other activities.

EXAMPLE

A stock solution of 300 pmoles of the standard inflammatory compound phorbol 12-myristate 13- acetate per 0.005 ml acetone was prepared. This solution was used to prepare four-fold dilutions of 20-deoxy-20-(2-hydroxyethylthio)-phorbol 12-myristate 13-acetate, prepared as in Example 3 of WO 87/07599 covering concentrations of the latter ranging from 4 to 64,000 pmoles per 0.005 ml. These solutions were used to demonstrate the anti-inflammatory activity of the latter compound by application of 0.005 ml to the insides of the right ears of mice, followed by the observation of ear inflammation/erythema during a 1-48 hour period. Inhibition of the phorbol 12-myristate 13-acetate induced inflammation was observed at the medium and higher concentrations of the inhibitor.

In a like manner, the anti-inflammatory activity of phorbol 12-myristate was demonstrated.

## Claims

1. A compound for medicine, said compound being of the formula (I):

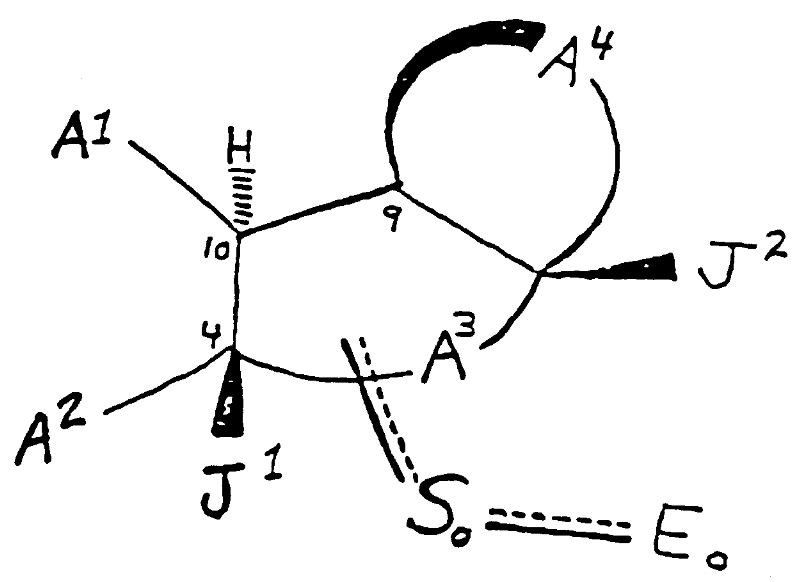

(I)

in the form of an individual isomer, an isomer mixture, a racemate or optical antipode, or a pharmaceutically acceptable salt thereof;

wherein

A1 and A2 may be individually selected from hydrogen and a straight chain or branched chain, cyclic or acyclic, saturated, unsaturated and/or aromatic carbon- and/or heteroatom-containing substituent having not more than 34 carbon atoms, not more than 24 halogen atoms and not more than 6 heteroatoms selected from oxygen, nitrogen and sulfur; or,

$A^1$ and $A^2$ taken together complete a 5- or 6-membered carbocyclic or heterocyclic ring, optionally substituted by one or more straight chain or branched chain, cyclic or acyclic, saturated, unsaturated, and/or aromatic carbon- and/or heteroatom-containing groups, which groups taken together contain a total of not more than 30 carbon atoms, not more than 24 halogen atoms and not more than 6 heteroatoms selected from oxygen, nitrogen, and sulfur;

$A^3$ is a three atom chain which completes a 7-membered carbocyclic ring optionally substituted by one or more straight chain or branched chain, cyclic or acyclic, saturated, unsaturated, and/or aromatic carbon- and/or heteroatom-containing groups, which groups taken methyl, ethyl, vinyl, ethynyl, propargyl, cyano, methoxy, ethoxy, trifluoromethyl, 2-hydroxyethyl, 2-hydroxypropyl, 3-hydroxypropyl, acetoxy, propanoyloxy, acetyl, propanoyl, hydroxyacetyl, 2-hydroxypropanoyloxy, 3-hydroxypropanoyl, acetamido, propanamido, hydroxyacetamido, 2-hydroxypropanamido, or 3-hydroxypropanamido, (each of which must be situated in the beta position), or $J^1$ taken together with A1, A2, or a ring formed by A1 together with A2 completes a 3- to 7-membered substituted or unsubstituted carbocyclic or heterocyclic ring;

J2 is selected from hydrogen, methyl, ethyl, hydroxymethyl, hydroxyethyl, vinyl, ethynyl, allyl, propargyl, n-propyl and iso-propyl;

$S_oE_o$ taken together comprise hydroxymethyl, 1-hydroxyethyl or 2-hydroxy-2-propyl.

2. A compound of the formula (I), as defined in claim 1, for use as an anti-inflammatory.

3. A compound for use according to claim 1 or claim 2 wherein the middle carbon atom of $A^3$ is substituted by SoEo.

4. A compound for use according to claim 3 which is a phorbol 12-ester.

5. A compound for use according to claim 4 which is selected from:

phorbol 12-[4-(9,10-dihydrophenanthrene-2)-butyrate];
phorbol 12-(2,4-difluorophenylacetate);
phorbol 12-[3,5-bis(trifluoromethyl) benzoate];
phorbol 12-[3,5-bis(trifluoromethyl)-phenylacetate;
phorbol 12-(4-n-hexylbenzoate);
phorbol 12-(3,5-dimethoxyphenylacetate);
phorbol 12-(4-phenylbenzoate); and
phorbol 12-myristate.

6. A compound of the formula (Ia);

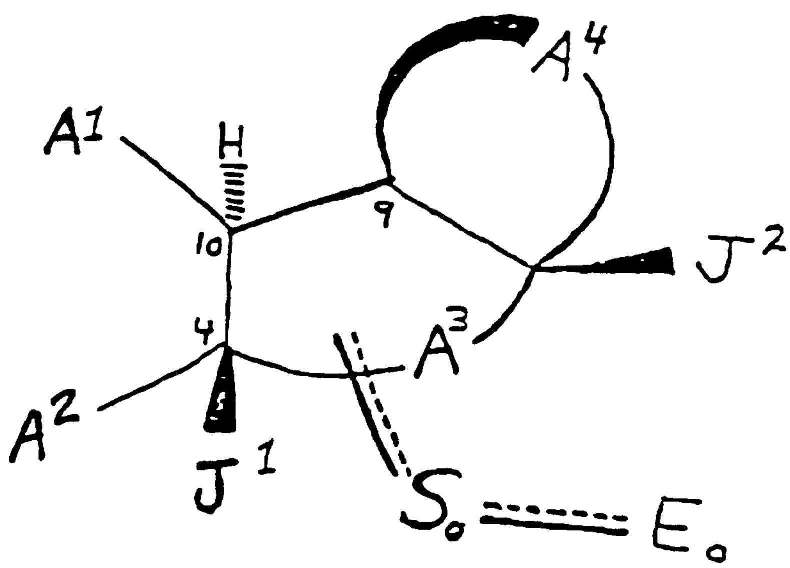

(Ia)

in the form of an individual isomer, an isomer mixture, a racemate or optical antipode, or a pharmaceutically acceptable salt thereof;
wherein

A1 and A2 may be individually selected from hydrogen and a straight chain or branched chain, cyclic or acyclic, saturated, unsaturated and/or aromatic carbon- and/or heteroatom-containing substituent having not more than 34 carbon atoms, not more than 24 halogen atoms and not more than 6 heteroatoms selected from oxygen, nitrogen and sulfur; or,

$A^1$ and $A^2$ taken together complete a 5- or 6-membered carbocyclic or heterocyclic ring, optionally substituted by one or more straight chain or branched chain, cyclic or acyclic, saturated, unsaturated, and/or aromatic carbon- and/or heteroatom-containing groups, which groups taken together contain a total of not more than 30 carbon atoms, not more than 24 halogen atoms and not more than 6 heteroatoms selected from oxygen, nitrogen, and sulfur;

$A^3$ is a three atom chain which completes a 7-membered carbocyclic ring optionally substituted by one or more straight chain or branched chain, cyclic or acyclic, saturated, unsaturated, and/or aromatic carbon- and/or heteroatom-containing groups, which groups taken together, excluding SoEo, contain not more than 7 carbon atoms, not more than 4 halogen atoms, and not more than 5 heteroatoms selected from oxygen, nitrogen and sulfur; provided that, excluding $S_oE_o$, the middle carbon atom of $A^3$ is not substituted by hydroxymethyl,

hydroxyethyl or 2-hydroxy-2-propyl.

$A^4$ completes a 6- membered carbocyclic ring (connected in the beta configuration to carbon atom 9), optionally substituted by one or more straight chain or branched chain cyclic or acyclic, saturated, unsaturated and/or aromatic carbon- and/or heteroatom-containing groups, linked to $A^4$ by single or double bonds, the group or groups optionally completing one or two additional rings by themselved and/or one or two additional rings by themselves and/or one to three additional rings when taken together with A1, A2, a ring formed by A1 and A2 together, and/or a bond to carbon atom 9, which groups taken together include not more than 40 carbon atoms, not more than 24 halogen atoms, and not more than 10 heteroatoms selected from oxygen, nitrogen, and sulfur; and wherein $A^4$ carries at least one substituent other than hydrogen hydroxy, beta-ethoxy, beta-benzoyloxy, beta-[2′-methyl-aminobenzoyloxy or Keto at $C_{12}$ and a hydroxy, amino, thiol, hydroxymethyl, mercaptomethyl, aminomethyl, 2-hydroxyethyl, carboxy, unsubstituted carboxamido, or unsubstituted aminocarbonyloxy group in the alpha configuration at $C_{13}$;

$J^1$ is selected from hydrogen, fluoro, chloro, hydroxy, amino, mono- or di-loweralkylamino.

methyl, ethyl, vinyl, ethynyl, propargyl, cyano, methoxy, ethoxy, trifluoromethyl, 2-hydroxyethyl, 2-hydroxypropyl, 3-hydroxypropyl, acetoxy, propanoyloxy, acetyl, propanoyl, hydroxyacetyl, 2-hydroxypropanoyloxy, 3-hydroxypropanoyl, acetamido, propanamido, hydroxyacetamido, 2-hydroxypropanamido, or 3-hydroxypropanamido, (each of which must be situated in the beta position), or $J^1$ taken together with A1, A2, or a ring formed by A1 together with A2 completes a 3- to 7-membered substituted or unsubstituted carbocyclic or heterocyclic ring;

J2 is selected from hydrogen, methyl, ethyl, hydroxymethyl, hydroxyethyl, vinyl, ethynyl, allyl, propargyl, n-propyl and iso-propyl;

$S_oE_o$ taken together comprise hydroxymethyl, 1-hydroxyethyl or 2-hydroxy-2-propyl; provided that if said compound is a phorbol 12-ester, it is not a phorbol 12-n-alkanoyl ester, a phorbol 12-alkenoyl ester, phorbol 12-[2′-methyl butanoate] or phorbol 12-(12′-N-dansylaminododecanoate).

7. A compound according to claim 6 wherein the middle carbon atom of $A^3$ is substituted by SoEo.

8. A compound of Claim 7 which is a phorbol 12-ester.

9. A compound of Claim 8 selected from the group consisting of:

i) phorbol 12-[4-(9,10-dihydrophenanthrene-2)-butyrate];
ii) phorbol 12-(2,4-difluorophenylacetate);
iii) phorbol 12-[3,5-bis(trifluoromethyl) benzoate];
iv) phorbol 12-[3,5-bis(trifluoromethyl)-phenylacetate;
v) phorbol 12-(4-n-hexylbenzoate);
vi) phorbol 12-(3,5- dimethoxypenylacetate); and
vii) phorbol 12-(4-phenylbenzoate).

10. The use of a compound of the formula (I) or (Ia), as defined in any one of the preceding claims, for the manufacture of a medicament for treating inflammatory conditions.

11. A pharmaceutical composition containing a compound of the formula (I) or (Ia) as defined in any one of the preceding claims, and a pharmaceutically acceptable carrier.

## Patentansprüche

1. Verbindung zur Verwendung in Medizin, wobei die Verbindung die Formel (I) :

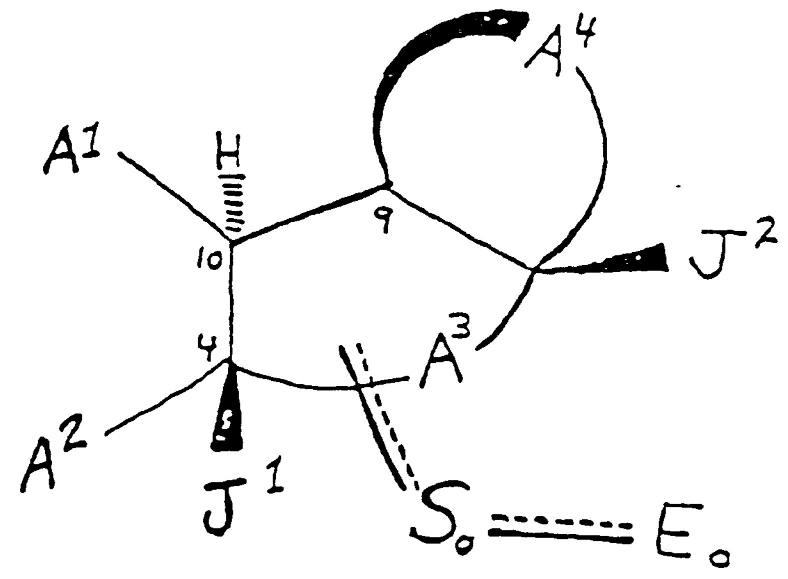

(I)

in der Form eines individuellen Isomeren, eines Isomerengemisches, eines Racemats oder optischen Antipoden oder eines pharmazeutisch akzeptablen Salzes von diesen hat,
wobei

A1 und A2 individuell ausgewählt werden können aus Wasserstoff und einem geradkettigen oder verzweigtkettigen, cyclischen oder acyclischen, gesättigten, ungesättigten und/oder aromatischen Kohlenstoff- und/oder heteroatomhaltingen Substituenten mit nicht mehr als 34 Kohlenstoffatomen, nicht mehr als 24 Halogenatomen und nicht mehr als 6 Heteroatomen, augewählt aus Sauerstoff, Stickstoff und Schwefel, oder

$A^1$ und $A^2$ zusammengenommen einen 5- oder 6-gliedrigen carbocyclischen oder heterocyclischen Ring vervollständigen, gegebenenfalls substituiert durch eine oder mehr geradkettige oder verzweigtkettige, cyclische oder acyclische, gesättigte, ungesättigte und/oder aromatische Kohlenstoff- und/oder heteroatomhaltige Gruppen, welche Gruppen zusammengenommen insgesamt nicht mehr als 30 Kohlenstoffatome, nicht mehr als 24 Halogenatome und nicht mehr als 6 Heteroatome, ausgewählt aus Sauerstoff, stickstoff und Schwefel, enthalten,

$A^3$ eine dreiatomige Kette ist, die einen siebengliedrigen carbocyclischen Ring vervollständigt, gegebenenfalls substituiert durch eine oder mehr geradkettige oder verzweigtkettige, cyclische oder acyclische, gesättigte, ungesättigte und/oder aromatische Kohlenstoff- und/oder heteroatomhaltige Gruppen, welche Gruppen zusammengenommen, unter Ausschluß von SoEo, nicht mehr als 7 Kohlenstoffatome, nicht mehr als 4 Halogenatome und nicht mehr als 5 Heteroatome, ausgewählt aus Sauerstoff, .Stickstoff und Schwefel, enthalten, vorausgesetzt, daß, unter Ausschluß von $S_oE_o$, das mittlere Kohlenstoffatom von $A^3$ nicht durch Hydroxymethyl, Hydroxyethyl oder 2-Hydroxy-2-Propyl substituiert ist,

$A^4$ einen 6-gliedrigen carbocyclischen Ring (verbunden in der Beta-Konfiguration mit Kohlenstoffatom 9) vervollständigt, ggf. ersetzt durch eine oder mehr geradkettige oder verzweigtkettige, cyclische oder acyclische, gesättigte, ungesättigte und/oder aromatische Kohlenstoff- und/oder heteroatomhaltige Gruppen, verbunden mit $A^4$ durch Einfach- oder Doppelbindungen, wobei die Gruppe oder Gruppen gegebenenfalls einen oder zwei zusätzliche Ringe durch sich selbst und/oder einen oder zwei zusätzliche Ringe durch sich selbst und/oder einen bis drei zusätzliche Ringe vervollständigen, wenn zusammengenommen mit A1, A2, einem von A1 und A2 zusammen gebildeten Ring und/oder einer Bindung mit Kohlenstoffatom 9, welche Gruppen zusammengenommen nicht mehr als 40 Kohlenstoffatome, nicht mehr als 24 Halogenatome und nicht mehr als 10 Heteroatome, ausgewählt aus Sauerstoff, Stickstoff und Schwefel, enthalten, und wobei $A^4$ zumindest einen Substituenten anders als Wasserstoff oder Hydroxy bei $C_{12}$ und eine Hydroxy-, Amino-, Thiol-, Hydroxymethyl-, Mercaptomethyl-, Aminomethyl-, 2-Hydroxyethyl-, Carboxy-, unsubstituierte Carboxamido- oder unsubstituierte Aminocarbonyloxy-Gruppe in der Alpha-Konfiguration bei $C_{13}$ trägt, $J^1$ ausgewählt ist aus Wasserstoff, Fluoro, Chloro, Hydroxy, Amino, Mono- oder Di-niederalkylamino, Methyl, Ethyl, Vinyl, Ethynyl, Propargyl, Cyano, Methoxy, Ethoxy, Trifluoromethyl, 2-Hydroxylethyl, 2-Hydroxypropyl, 3-Hydroxylpropyl, Acetoxy, Propanoyloxy, Acetyl, Propanoyl, Hydroxyacetyl, 2-Hydroxypropanoyloxy, 3-Hydroxypropanoyl, Acetamido, Propanamido, Hydroxyacetamido, 2-Hydroxypropanamido, oder 3-Hydroxypropanamido (die jeweils in der Beta-Position liegen müssen) oder $J^1$ zusammengenommen mit A1, A2 oder einem von A1 zusammen mit A2 gebildeten Ring einen 3- bis 7-gliedrigen substituierten oder unsubstituierten carbocyclischen oder heterocyclischen Ring vervollständigt,

J2 ausgewählt ist aus Wasserstoff, Methyl, Ethyl, Hydroxymethyl, Hydroxyethyl, Vinyl, Ethynyl, Allyl, Propargyl, n-Propyl und Iso-Propyl,

$S_oE_o$ zusammengenommen Hydroxymethyl, 1-Hydroxyethyl oder 2-Hydroxy-2-Propyl umfassen.

2. Verbindung der Formel (I) nach Anspruch 1, zur Verwendung als entzündungshemmendes Mittel.

3. Verbindung zur Verwendung nach Anspruch 1 oder Anspruch 2, bei der das mittlere Kohlenstoffatom von $A^3$ durch SoEo ersetzt ist.

4. Verbindung zur Verwendung nach Anspruch 3, welche ein Phorbol-12-Ester ist.

5. Verbindung zur Verwendung nach Anspruch 4, die ausgewählt ist aus:

Phorbol-12-[4-(9,10-dihydrophenanthren-2)-butyrat],
Phorbol-12-2(2,4-difluorophenylacetat),
Phorhol-12-(3,5-bis(trifluoromethyl)-benzoat],
Phorbol-12-[3,5-bis(trifluoromethyl)-phenylacetat,
Phorbol-12- 4-n-hexylbenzoat),
Phorbol-12-(3,5-dimethoxyphenylacetat),
Phorbol-12-(4-Phenylbenzoat), und
Phorbol-12-Myristat.

6. Verbindung der Formel (Ia):

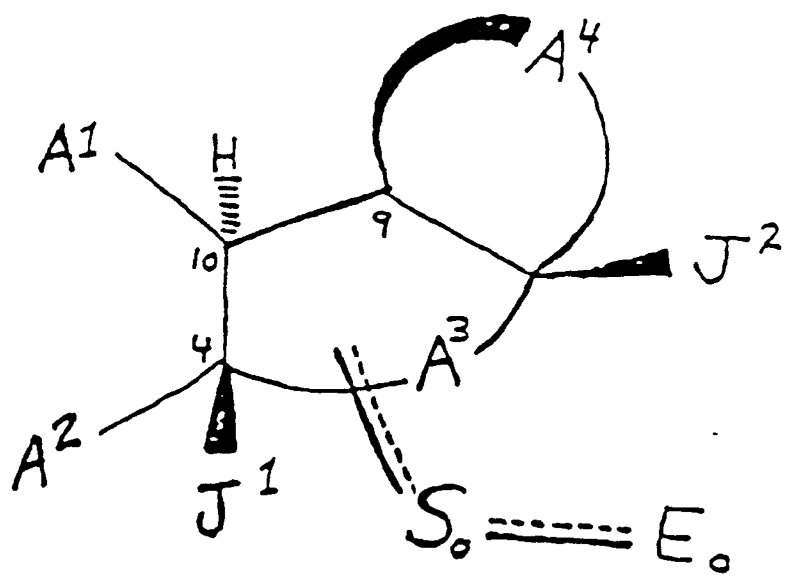

(Ia)

in der Form eines individuellen Isomeren, eines Isomerengemisches, eines Racemats oder optischen Antipoden oder eines pharmazeutisch akzeptablen Salzes von diesen hat,

wobei

A1 und A2 individuell ausgewählt werden können aus Wasserstoff und einem geardkettigen oder verzweigtkettigen, cyclischen oder acyclischen, gesättigten, ungesättigten und/oder aromatischen Kohlenstoff- und/oder heteroatomhaltingen Substituenten mit nicht mehr als 34 Kohlenstoffatomen, nicht mehr als 24 Halogenatomen und nicht mehr als 6 Heteroatomen, augewählt aus Sauerstoff, Stickstoff und Schwefel, oder

$A^1$ und $A^2$ zusammengenommen einen 5- oder 6-gliedrigen carbocyclischen oder heterocyclischen Ring vervollständigen, gegebenenfalls substituiert durch eine oder mehr geradkettige oder verzweigtkettige, cyclische oder acyclische, gesättigte, ungesättigte und/oder aromatische Kohlenstoff- und/oder heteroatomhaltige Gruppen, welche Gruppen zusammengenommen insgesamt nicht mehr als 30 Kohlenstoffatome, nicht mehr als 24 Halogenatome und nicht mehr als 6 Heteroatome, ausgewählt aus Sauerstoff, Stickstoff und schwWefel, enthalten,

$A^3$ eine dreiatomige Kette ist, die einen siebengliedrigen carbocyclischen Ring vervollständigt, gegebenenfalls substituiert durch eine oder mehr geradkettige oder verzweigtkettige, cyclische oder acyclische, gesättigte, ungesättigte und/oder aromatische Kohlenstoff- und/oder heteroatomhaltige Gruppen, welche Gruppen zusammengenommen, unter Ausschluß von SoEo, nicht mehr als 7 Kohlenstoffatome, nicht mehr als 4 Halogenatome und nicht mehr als 5 Heteroatome, ausgewählt aus Sauerstoff, Stickstoff und Schwefel, enthalten, vorausgesetzt, daß, unter Ausschluß von $S_oE_o$, das mittlere Kohlenstoffatom von $A^3$ nicht durch Hydroxymethyl, Hydroxyethyl oder 2-Hydroxy-2-Propyl substituiert ist,

$A^4$ einen 6-gliedrigen carbocyclischen Ring (verbunden in der Beta-Konfiguration mit Kohlenstoffatom 9) vervollständigt, ggf. ersetzt durch eine oder mehr geradkettige oder verzweigtkettige, cyclische oder acyclische, gesättigte, ungesättigte und/oder aromatische Kohlenstoff- und/oder heteroatomhaltige Gruppen, verbunden mit $A^4$ durch Einfach- oder Doppelhindungen, wobei die Gruppe oder Gruppen gegebenenfalls einen oder zwei zusätzliche Ringe durch sich selbst und/oder einen oder zwei zusätzliche Ringe durch sich selbst und/oder einen his drei zusätzliche Ringe vervollständigen, wenn zusammengenommen mit A1, A2, einem von A1 und A2 zusammen gebildeten Ring und/oder einer Bindung mit Kohlenstoffatom 9, welche Gruppen zusammengenommen nicht mehr als 40 Kohlenstoffatome, nicht mehr als 24 Halogenatome und nicht mehr als 10 Heteroatome, ausgewählt aus Sauerstoff, Stickstoff und Schwefel, enthalten, und wobei $A^4$ zumindest einen Substituenten anders als Wasserstoff, Hydroxy, Beta-Ethoxy, Beta-Benzoyloxy, Bety- 2′-Methyl-Aminobenzoyloxy oder Keto bei $C_{12}$ und eine Hydroxy-, Amino-, Thiol-, Hydroxymethyl-, Mercaptomethyl-, Aminomethyl-, 2-Hydroxyethyl-, Carboxy-, unsubstituierte Carboxamido- oder unsubstituierte Aminocarbonyloxy-Gruppe in der Alpha-Konfiguration bei $C_{13}$ trägt, $J^1$ ausgewählt ist aus Wasserstoff, Fluoro, Chloro, Hydroxy, Amino, Mono- oder Di-niederalkylamino, Methyl, Ethyl, Vinyl, Ethynyl, Propargyl, Cyano, Methoxy, Ethoxy, Trifluoromethyl, 2-Hydroxylethyl, 2-Hydroxypropyl, 3-Hydroxylpropyl, Acetoxy, Propanoyloxy, Acetyl, Propanoyl, Hydroxyacetyl, 2-Hydroxypropanoyloxy, 3-Hydroxypropanoyl, Acetamido, Propanamido, Hydroxyacetamido, 2-Hydroxypropanamido, oder 3-Hydroxypropanamido (die jeweils in der Beta-Position liegen müssen) oder $J^1$ zusammengenommen mit A1, A2 oder einem von A1 zusammen mit A2 gebildeten Ring einen 3- bis 7-gliedrigen substituierten oder unsubstituierten carbocyclischen oder heterocyclischen Ring vervollständigt,

J2 ausgewählt ist aus Wasserstoff, Methyl, Ethyl, Hydroxymethyl, Hydroxyethyl, Vinyl, Ethynyl, Allyl, Propargyl, n-Propyl und Iso-Propyl,

$S_oE_o$ zusammengenommen Hydroxymethyl, 1-Hydroxyethyl oder 2-Hydroxy-2-Propyl umfassen, vorausgesetzt, daß, falls die Verbindung ein Phorbol-12-Ester ist, sie nicht ein Phorbol-12-n-Alkanoyl-Ester, ein Phorbol- 12-Alkenoyl-Ester, Phorbol-12- 2′-Methyl-Butanoat oder Phorbol-12-(12′-N-Dansylaminododecanoat) ist.

7. Verbindung nach Anspruch 6, bei der das mittlere Kohlenstoffatom von $A^3$ durch SoEo substituiert ist.

8. Verbindung nach Anspruch 7, welche ein Phorbol-12-Ester ist.

9. Verbindung nach Anspruch 8, ausgewählt aus der aus

i) Phorbol-12-[4-(,10-Dihydrophenanthren-2)-butyrat],
ii) Phorbol-12-(2,4-difluorophenylacetat),
iii) Phorbol-12-[3,5-bis(trifluoromethyl)-benzoat],
iv) Phorbol-12-[3,5-bis(trifluoromethyl)-phenylacetat,
v) Phorbol-12-(4-n-hexylbenzoat),
vi) Phorbol-12-(3,5-dimethoxyphenylacetat) und
vii) Phorbol-12-(4-phenylbenzoat) bestehenden Gruppe.

10. Verwendung einer Verbindung der Formel (I) oder (Ia) nach einem der vorhergehenden Ansprüche zur Herstellung eines Medikaments zur Behandlung von Entzündungszuständen.

11. Pharmazeutische Zusammensetzung, die eine Verbindung der Formel (I) oder (Ia) nach einem der vorhergehenden Ansprüche und einen pharmazeutisch akzeptablen Träger enthält.

## Revendications

1. Composé à usage médical, sous la forme d'un isomère individuel, d'un mélange d'isomeres, d'un racémate ou d'un antipode optique, ou sel pharmaceutiquement acceptable de ce composé, lequel répond à la formule (I)

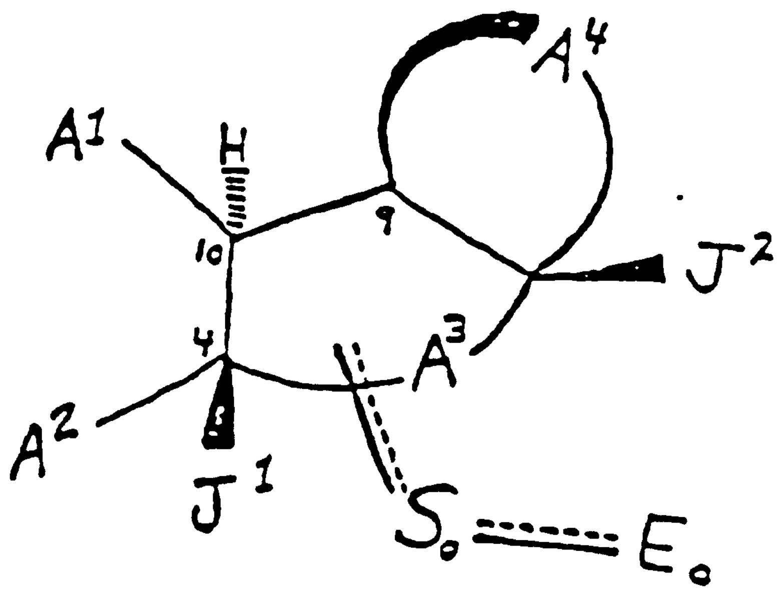

(I)

dans laquelle

$A^1$ et $A^2$ peuvent être choisis individuellement parmi des atomes d'hydrogene et des substituants à chaine droite ou à chaine ramifiee, cycliques ou acycliques, satures, insatures et/ou aromatiques contenant des atomes de carbone et/ou des hétéroatomes, ne renfermant pas plus de 34 atomes de carbone, pas plus de 24 atomes d'halogènes et pas plus de 6 hétéroatomes choisis parmi les atomes d'oxygène, d'azote et de soufre; ou

$A^1$ et $A^2$, pris ensemble, complètent un noyau carbocyclique ou hétérocyclique penta- ou hexagonal, éventuellement substitué par un ou plusieurs groupements à chaîne droite ou ramifiée, cycliques ou acycliques, saturés, insaturés et/ou aromatiques contenant des atomes de carbone et/ou des hétéroatomes, lesquels groupements, pris ensemble, ne contiennent au total pas plus de 30 atomes de carbone, pas plus de 24 atomes d'halogènes et pas plus de 6 hétéroatomes choisis parmi les atomes d'oxygène, d'azote et de soufre;

$A^3$ est une chaîne de 3 atomes qui complète un noyau carbocyclique heptagonal, éventuellement substitué par un ou plusieurs groupements à chaîne droite ou ramifiée, cycliques ou acycliques, saturés, insaturés et/ou aromatiques contenant des atomes de carbone et/ou des hétéroatomes, lesquels groupements, pris ensemble à l'exclusion de $S_oE_o$, ne contiennent pas plus de 7 atomes de carbone, pas plus de 4 atomes d'halogènes et pas plus de 5 hétéroatomes choisis parmi les atomes d'oxygène, d'azote et de soufre; étant spécifé qu'à l'exclusion de $S_oE_o$, l'atome de carbone du milieu de $A^3$ n'est pas substitué par un reste hydroxyméthyle, hydroxyethyle ou 2-hydroxy-2-propyle;

$A^4$ complète un noyau carbocyclique hexagonal (relie dans la configuration β à l'atome de carbone 9), éventuellement substitué par un ou plusieurs groupements à chaîne droite ou ramifiée, cycliques ou acycliques, saturés, insatures et/ou aromatiques contenant des atomes de carbone et/ou des hétéroatomes, reliés à $A^4$ par des liaisons simples ou doubles, ce ou ces groupements complétant éventuellement un ou deux noyaux supplémentaires par eux-mêmes et/ou un ou deux noyaux supplémentaires par eux-mêmes et/ou un à trois noyaux supplémentaires lorsqu'ils sont pris en même temps que $A^1$, $A^2$, un noyau forme par $A^1$ et $A^2$ ensemble et/ou une liaison à l'atome de carbone 9, lesquels groupements, pris ensemble, ne contiennent pas plus de 40 atomes de carbone, pas plus de 24 atomes d'halogènes et pas plus de 10 hétéroatomes choisis parmi les atomes d'oxygène, d'azote et de soufre; et $A^4$ porte, en $C_{12}$, au moins un substituant autre qu'un atome d'hydrogène ou un radical hydroxy et, dans la configuration α en $C_{13}$, un groupement hydroxy, amino, thiol, hydroxyméthyle, mercaptométhyle, aminométhyle, 2-hydroxyéthyle, carboxy, carboxamido non substitué ou aminocarbonyloxy non substitué;

$J^1$ est choisi parmi un atome d'hydrogène, de fluor, de chlore, un radical hydroxy, amino, mono- ou dialkyle inférieur-amino, méthyle, éthyle, vinyle, éthynyle, propargyle, cyano, méthoxy, éthoxy, trifluorométhyle, 2-hydroxyéthyle, 2-hydroxypropyle, 3-hydroxypropyle, acétoxy, propanoyloxy, acétyle, propanoyle, hydroxyacétyle, 2-hydroxypropanoyloxy, 3-hydroxypropanoyle, acétamido, propanamido, hydroxyacétamido, 2-hydroxypropanamido ou 3-hydroxypropanamido (dont chacun doit être situé en position β), ou $J^1$, pris en même temps que $A^1$, $A^2$ ou un noyau formé par $A^1$ et $A^2$ ensemble, complète un noyau carbocyclique ou hétérocyclique à 3-7 chaînons, substitué ou non substitue;

$J^2$ est choisi parmi un atome d'hydrogène et les radicaux méthyle, éthyle, hydroxyméthyle,

hydroxyethyle, vinyle, ethynyle, allyle, propargyle, n-propyle et isopropyle;

$S_oE_o$, pris ensemble, constituent un groupement hydroxyméthyle, 1-hydroxyethyle ou 2-hydroxy-2-propyle.

2. Compose de formule (I) selon la revendication 1, à usage comme anti-infammatoire.

3. Composé à usage selon la revendication 1 ou 2, dans lequel l'atome de carbone du milieu de $A^3$ est substitué par $S_oE_o$.

4. Composé à usage selon la revendication 3, qui est un phorbol-12-ester.

5. Composé à usage selon la revendication 4, qui est choisi parmi les suivants:

Phorbol-12-[4-(9,10-dihydrophénanthrène-2)-butyrate];
Phorbol-12-(2,4-difluorophénylacétate);
Phorbol-12-[3,5-bis(trifluorométhyl)-benzoate];
Phorbol-12-[3,5-bis(trifluorométhyl)-phénylacétate];
Phorbol-12-(4-n-hexylbenzoate);
Phorbol-12-(3,5-diméthoxyphénylacétate);
Phorbol-12-(4-phénylbenzoate); et
Phorbol-12-myristate.

6. Composé à usage médical, sous la forme d'un isomère individuel, d'un mélange d'isomères, d'un racémate ou d'un antipode optique, ou sel pharmaceutiquement acceptable de ce composé, lequel répond à la formule (Ia)

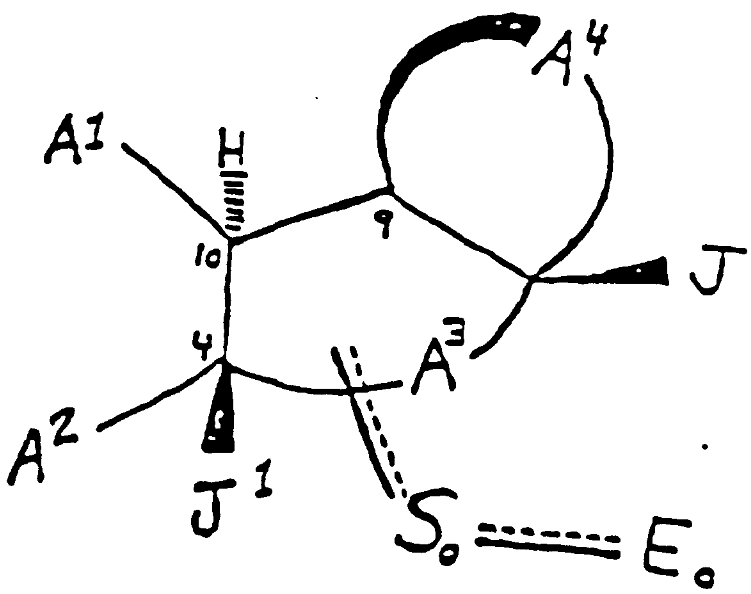

(Ia)

dans laquelle

$A^1$ et $A^2$ peuvent être choisis individuellement parmi des atomes d'hydrogène et des substituants à chaîne droite ou à chaîne ramifiée, cycliques ou acycliques, saturés, insaturés et/ou aromatiques contenant des atomes de carbone et/ou des hétéroatomes, ne renfermant pas plus de 34 atomes de carbone, pas plus de 24 atomes d'halogènes et pas plus de 6 hétéroatomes choisis parmi les atomes d'oxygène, d'azote et de soufre; ou

$A^1$ et $A^2$, pris ensemble, complètent un noyau carbocyclique ou hétérocyclique penta- ou hexagonal, éventuellement substitué par un ou plusieurs groupements à chaîne droite ou ramifiée, cycliques ou acycliques, saturés, insaturés et/ou aromatiques contenant des atomes de carbone et/ou des hétéroatomes, lesquels groupements, pris ensemble, ne contiennent au total pas plus de 30 atomes de carbone, pas plus de 24 atomes d'halogènes et pas plus de 6 hétéroatomes choisis parmi les atomes d'oxygène, d'azote et de soufre;

$A^3$ est une chaîne de 3 atomes qui complète un noyau carbocyclique heptagonal, éventuellement substitué par un ou plusieurs groupements à chaîne droite ou ramifiée, cycliques ou acycliques, saturés, insaturés et/ou aromatiques contenant des atomes de carbone et/ou des hétéroatomes, lesquels groupements, pris ensemble à l'exclusion de $S_oE_o$, ne contiennent pas plus de 7 atomes de carbone, pas plus de 4 atomes d'halogènes et pas plus de 5 hétéroatomes choisis parmi les atomes d'oxygène, d'azote et de soufre; étant specife qu'à l'exclusion de SoEo, l'atome de carbone du milieu de $A^3$ n'est pas substitue par un reste hydroxymethyle, hydroxyethyle ou 2-hydroxy-2-propyle;

$A^4$ complète un noyau carbocyclique hexagonal (relie dans la configuration β à l'atome de carbone 9), éventuellement substitué par un ou plusieurs groupements à chaîne droite ou ramifiée, cycliques ou acycliques, saturés, insaturés et/ou aromatiques contenant des atomes de carbone et/ou des hétéroatomes, reliés à $A^4$ par des liaisons simples ou doubles, ce ou ces groupements complétant éventuellement un ou deux noyaux supplémentaires par eux-mêmes et/ou un ou deux noyaux supplémentaires par eux-mêmes et/ou à trois noyaux supplémentaires lorsqu'ils sont pris en même temps que $A^1$, $A^2$, un noyau formé par $A^1$ et $A^2$ ensemble et/ou une liaison à l'atome de carbone 9, lesquels groupements, pris ensemble, ne contiennent pas plus de 40 atomes de carbone, pas plus de 24 atomes d'halogènes et pas plus de 10 hétéroatomes choisis parmi les atomes d'oxygène, d'azote et de soufre; et $A^4$ porte, en $C_{12}$, au moins un substituant autre qu'un atome d'hydrogène, un radical hydroxy, β-hydroxy, β-benzoyloxy, β-[2′-méthylaminobenzoyloxy] ou céto et, dans la configuration α en $C_{13}$, un groupement hydroxy, amino, thiol, hydroxyméthyle, mercaptométhyle, aminométhyle, 2-hydroxyéthyle, carboxy, carboxamido non substitué ou aminocarbonyloxy non substitué;

$J^1$ est choisi parmi un atome d'hydrogène, de fluor, de chlore, un radical hydroxy, amino, mono- ou dialkyle inférieur-amino, méthyle, éthyle, vinyle, éthynyle, propargyle, cyano, méthoxy, éthoxy, trifluoromethyle, 2-hydroxyéthyle, 2-hydroxypropyle, 3-hydroxypropyle, acetoxy, propanoyloxy, acetyle, propanoyle, hydroxyacetyle, 2-hydroxypropanoyloxy, 3-hydroxypropanoyle, acetamido, propanamido, hydroxyacétamido, 2-hydroxypropanamido ou 3-hydroxypropanamido (dont chacun doit être situé en position β), ou $J^1$, pris en même temps que $A^1$, $A^2$ ou un noyau forme par $A^1$ et $A^2$ ensemble, complete un noyau carbocyclique ou heterocyclique a 3-7 chainons, subsitué ou non substitue;

$J^2$ est choisi parmi un atome d'hydrogene et les radicaux methyle, ethyle, hydroxymethyle, hydroxyethyle, vinyle, éthynyle, allyle, propargyle, n-propyle et isopropyle;

SoEo, pris ensemble, constituent un groupement hydroxyméthyle, 1-hydroxyéthyle ou 2-hydroxy-2-propyle;

étant spécifié que si ledit composé est un phorbol-12-ester, il n'est pas un phorbol-12-n-alcanoyl-ester, un phorbol-12-alcénoyl-ester, un phorbol-12-[2′-méthylbutanoate] ou un phorbol-12-(12′-N-dansylaminododécanoate).

7. Composé selon la revendication 6, dans lequel l'atome de carbone du milieu de $A^3$ est substitué par SoEo.

8. Composé selon la revendication 7, qui est un phorbol-12-ester.

9. Composé selon la revendication 8, choisi dans le groupe constitué par:

i) Phorbol-12-[4-(9,10-dihydrophénanthrène-2)-butyrate];
ii) Phorbol-12-(2,4-difluorophénylacétate);
iii) Phorbol-12-[3,5-bis(trifluorométhyl)-benzoate];
iv) Phorbol-12-[3,5-bis(trifluorométhyl)-phénylacétate];
v) Phorbol-12-(4-n-hexylbenzoate);
vi) Phorbol-12-(3,5-dimethoxyphénylacétate); et
vii) Phorbol-12-(4-phénylbenzoate).

10. Utilisation d'un compose de formule (I) ou (Ia) selon l'une quelconque des revendications 1 à 9, pour la preparation d'un medicament pour le traitement d'etats inflammatoires.

11. Composition pharmaceutique contenant un compose de formule (I) ou (Ia) selon l'une quelconque des reendications 1 à 9 et un excipient pharmaceutiquement acceptable.